# EUROPEAN PATENT APPLICATION

(11) **EP 2 338 992 A2**
(43) Date of publication of application: **29.06.2011**
(21) Application number: 10179177.0
(22) Date of filing: 29.08.2006
(51) Int. Cl.: C12N 15/11, A61K 31/711, C07H 19/06, C07H 19/16

(54) **Antisense compounds having enhanced anti-microRNA activity**

(30) Priority: 29.08.2005 US 712211 P
(62) Divisional of application: 06802706.9
(71) Applicant: Regulus Therapeutics, Inc, Carlsbad, CA 92008 (US)
(72) Inventor: Esau, Christine, La Jolla, CA 92037 (US); Swayze, Eric E., Encinitas, CA 92024 (US)
(74) Representative: Warner, James Alexander

(57) **Abstract**

Antisense compounds, compositions and methods are provided for modulating the levels expression, processing and function of miRNAs. The antisense compounds exhibit enhanced anti-miRNA activity. Further provided are methods for enhancing the inhibitory activity of an antisense compound targeting a miRNA, comprising incorporating stability enhancing sugar modifications into the antisense compounds.

## Description

### Cross-reference to Related Applications

This application claims priority under 35 U.S.C. § 119(e) to U.S. Provisional Application Serial No. 60/712,211 filed August 29, 2045,

### Field of the Invention

The present invention provides compositions and methods for modulation of small non-coding RNAs, including miRNA. In particular, this invention relates to antisense compounds, particularly antisense compounds having chemically modified nucleosides arranged in patterns which in some embodiments, enhance the ability of the antisense compounds to hybridize with or sterically occlude small non-coding RNA targets, particularly miRNAs.

### Background of the Invention

MicroRNAs (miRNAs) are small (approximately 21-24 nucleotides in length, these are also known as "mature" miRNAs), non-coding RNA molecules encoded in the genomes of plants and animals. These highly conserved, endogenously expressed RNAs are believed to regulate the expression of genes by binding to the 3'-untranslated regions (3'-UTR) of specific mRNAs. MiRNAs may act as key regulators of cellular processes such as cell proliferation, cell death (apoptosis), metabolism, and cell differentiation. On a larger scale, miRNA expression has been implicated in early development, brain development and disease progression (such as cancers and viral infections). There is speculation that in higher eukaryotes, the role of miRNAs in regulating gene expression could be as important as that of transcription factors. More than 200 different miRNAs have been identified in plants and animals (Ambros et al., Curr. Biol., 2003, 13, 807-818). Mature miRNAs appear to originate from long endogenous primary miRNA transcripts (also known as pri-miRNAs, pri-mirs, pri-miRs or pri-pre-miRNAs) that are often hundreds of nucleotides in length (Lee, et al., EMBO J., 2002, 21(17), 4663-4670).

Functional analyses of miRNAs have revealed that these small non-coding RNAs contribute to different physiological processes in animals, including developmental timing, organogenesis, differentiation, patterning, embryogenesis, growth control and programmed cell death. Examples of particular processes in which miRNAs participate include stem cell differentiation, neurogenesis, angiogenesis, hematopoiesis, and exocytosis (reviewed by Alvarez-Garcia and Miska, Development, 2005, 132, 4653-4662).

### Cross-reference to Related Applications

This application claims priority under 35 U.S.C. § 119(e) to U.S. Provisional Applications Serial No. 60/712,211 filed August 29, 2005,

### Field of the Invention

The present invention provides compositions and methods for modulation of small non-coding RNAs, including miRNA. In particular, this invention relates to antisense compounds, particularly antisense compounds having chemically modified nucleosides arranged in patterns which in some embodiments, enhance the ability of the antisense compounds to hybridize with or sterically occlude small non-coding RNA targets, particularly miRNAs.

### Background of the Invention

MicroRNAs (miRNAs) are small (approximately 21-24 nucleotides in length, these are also known as "mature" miRNA), non-coding RNA molecules encoded in the genomes of plants and animals. These highly conserved, endogenously expressed RNAs are believed to regulate the expression of genes by binding to the 3'-untranslated regions (3'-UTR) of specific mRNAs. MiRNAs may act as key regulators of cellular processes such as cell proliferation, cell death (apoptosis), metabolism, and cell differentiation. On a larger scale, miRNA expression has been implicated in early development, brain development and disease progression (such as cancers and viral infections). There is speculation that in higher eukaryotes, the role of miRNAs in regulating gene expression could be as important as that of transcription factors. More than 200 different miRNAs have been identified in plants and animals (Ambros et al., Curr. Biol., 2003, 13, 807-818). Mature miRNAs appear to originate from long endogenous primary miRNA transcripts (also known as pri-miRNAs, pri-mirs, pri-miRs or pri-pre-miRNAs) that are often hundreds of nucleotides in length (Lee, et al., EMBO J., 2002, 21(17), 4663-4670).

Functional analyses of miRNAs have revealed that these small non-coding RNAs contribute to different physiological processes in animals, including developmental timing, organogenesis, differentiation, patterning, embryogenesis, growth control and programmed cell death. Examples of particular processes in which miRNAs participate include stem cell differentiation, neurogenesis, angiogenesis, hematopoiesis, and exocytosis (reviewed by Alvarez-Garcia and Miska, Development, 2005, 132, 4653-4662).

MiRNAs are thought to exercise post-t-ranscriptional control in most eukaryotic organisms and have been detected in plants and animals as well as certain viruses. A large number of miRNAs have been identified from several species (see for example PCT Publication WO 03/029459 and Published US Patent Applications 20050222399, 20050227934, 20050059005 and 20050221293, each of which are incorporated herein by reference in their entirety) and many more have been bioinformatically predicted. Many of these miRNA are conserved across species, but species specific miRNA have also been identified (Pillai, RNA, 2005,11,1753-1761).

Consequently, there is a need for agents that regulate gene expression via the mechanisms mediated by small non-coding RNAs. Compounds that can increase or decrease gene expression or activity by modulating the levels of miRNA in a cell with greater activity than unmodified oligonucleotides are therefore desirable.

The present invention therefore provides enchanced chemically modified antisense compounds which are useful for modulating the levels, activity, or function of miRNAs. One having skill in the art, once armed with this disclosure will be able, without undue experimentation, to identify compounds, compositions and methods for these uses.

### Summary of the Invention

The present invention provides antisense compounds comprising a plurality of nucleosides with substituted or unsubstituted 2'-O-alkyl modified nucleosides and a plurality of nucleosides with bicyclic sugar modified nucleosides. Further, each of said substituted or unsubstituted 2'-O-alkyl modified nucleosides have the same sugar modification and each of said bicyclic sugar modified nucleosides have the same bicyclic modification. Each of the 2'-substituent group each of said substituted or unsubstituted 2'-O-alkyl modified nucleosides is, independently, -O-(CH₂)ⱼ-CH₃, -O-(CH₂)₂-O-CH₃, -O(CH₂)₂-S-CH₃, O-(GH₂)₂-O-N(Rₘ)(Rₙ) or O-CH₂-C(=O)-N(Rₘ)(Rₙ), where j is 0, 1 or 2 and each Rₘ and Rₙ is, independently, H, an amino protecting group or substituted or unsubstituted C1-C10 alkyl. The bicyclic sugar of each bicyclic sugar modified nucleoside comprises a 2'-O-CH₂-4', or a 2'-O-(CH₂)₂-4' bridge.

Additionally, the antisense compounds provided herein comprise linked substituted or unsubstituted 2'-O-alkyl modified nucleosides having at least two internal regions of bicyclic sugar modified nucleosides wherein each internal region comprises from 1 to 4 bicyclic sugar modified nucleosides. The antisense compound may comprise from 3 to about 7 internal regions of bicyclic sugar modified nucleosides. Each internal region of bicyclic sugar modified nucleosides may be flanked by from 1 to about 8 substituted or unsubstituted 2'-O-alkyl modified nucleosides.

The antisense compounds may have one of the following formulas: A₅-B₁-A₅-B₁-A₄-B₁-A₆, (A-A-B)₇(-A)₂, (A-A-A-B)₅-A₃, A₅-B₁-A₂-B₁-A₂-B₁-A₂-B₁-A₁-B₁-A₃-B1-A₂, A₃-B₃-A₂-B₃-A₂-B₃-A₇, A₃-B₃-A₂-B₃-A₂-B₃-A₂-B₃-A₂, A₃-B₂-A₂-B₃-A₂-B₂-A₈, or A₅-B₂-A₂-B₃-A₂-B₃-A₅, wherein A is a substituted or unsubstituted 2'-O-alkyl modified nucleosides, B is a bicyclic sugar modified nucleoside, and each subscript number represents the number of repeats of the preceding nucleoside or block of nucleosides. Further, each 2'-substituent group of said substituted or unsubstituted 2'-O-akyl modified nucleosides is -O-(CH₂)₂-O-CH₃ and each bicyclic modified nucleoside comprises a 2'-O-CH₂-4'bridge.

The antisense compounds provided herein comprise from about 15 to about 30 linked nucleosides.

Further, each internucleoside linking group of the antisense compounds provided herein is, independently, a phosphodiester or a phosphorothioate. The antisense compounds further comprise a plurality of phosphorothioate internucleoside linkages.

Also provided are antisense compounds as described, further comprising one or more regions of from 1 to 4 differentially modified nucleosides wherein said differentially modified nucleosides are different from the other nucleosides in said antisense compound. The differentially modified nucleosides are 2'-deoxynucleosides.

The present invention provides a method of enhancing the ability of a substituted or unsubstituted 2'-O-allcyl uniformly modified antisense compound to modulate the activity of a miRNA by incorporating into said antisense compound a plurality of bicyclic sugar modified nucleosides. The 2'-substituent group each of said substituted or unsubstituted 2'-O-alkyl modified nucleosides is, independently, -O-(CH₂)ⱼ-CH₃, -O-(CH₂)₂-O-CH₃, -O(CH₂)₂-S-CH₃, O-(CH₂)₂-ON(Rₘ)(Rₙ) or O-CH₂-C(=O)-N(Rₘ)(Rₙ), where j is 0, 1 or 2 and each Rₘ and Rₙ is, independently, H, an amino protecting group or substituted or unsubstituted C1-C10 alkyl. The bicyclic sugar of each bicyclic sugar modified nucleoside comprises a 2'-O-CH₂-4', or a 2'-O-(CH₂)₂-4' bridge.

### Detailed Description

Antisense compounds have been widely used to target coding genes, for the purposes of elucidating gene function, and additionally for therapeutic applications. Antisense compounds typically have chemically modified nucleosides arranged in patterns that elicit target cleavage by cellular enzymes such as RNase H or by enzymes of cellular complexes such as RISC. Alternatively, antisense compounds have chemically modified nucleosides arranged in patterns that promote steric hinderance (or steric occlusion) of the target RNA (e.g. for modulating splicing). The same chemically modified motifs used to target mRNA have not been found to be particularly effective when targeting small non-coding RNAs, such as miRNAs. Antisense compounds which contain uniform modifications have been shown to be active in inhibiting the activity of small non-coding RNAs, such as miRNAs, but there exists a need to find antisense compounds with enhanced activity to inhibit small non-coding RNA, particularly miRNAs, compared to the uniformly modified antisense compounds.

It has been found that the use of chemically modified nucleosides in an antisense compound can affect the ability of the antisense compound to bind to, and modulate, and target small non-coding RNA, such as miRNA. It has further been discovered that the arrangement of chemically modified nucleosides in an antisense compound also affects the ability of the antisense compound to bind to, and modulate, a targeted small non-coding RNA such as miRNA. The present invention provides antisense compounds with enhanced activity for use in the modulation of small non-coding RNA such as miRNA. Further provided are methods for enhancing the activity of an otherwise uniformly modified antisense compound by incorporating nucleotides comprising a second, distinct chemical modification. Such enhanced activity is particularly useful for modulation of small non-coding RNA such as miRNA *in vivo.* In a prefered embodiment, the small non-coding RNA to be modulated with the compounds of the instant invention are miRNA, but the compounds may be useful to modulate the activity of other small non-coding RNA as well. In one embodiment the antisense compounds comprise a plurality of substituted or unsubstituted 2'-O-alkyl modified nucleotides (e.g. 2'-O-Methyl or 2'-methoxyethoxy) and a plurality of bicyclic modified nucleotides (e.g. LNA™ or ENA™). In a further embodiment antisense compounds which are uniformly modified with substituted or unsubstituted 2'-O-alkyl modified nucleotides are enhanced by replacing a plurality of the substituted or unsubstituted 2'-O-alkyl modified nucleotides with nucleotides containing a bicyclic sugar moiety. In a further embodiment, at least 25% of the internucleoside linkages are modified to resist nuclease cleavage (e.g. phosphorothioate modified internucleoside linkages). In another embodiment, at least 50% of the internucleoside linkages are modified to resist nuclease cleavage.

The present invention provides antisense compounds which are capable of hybridizing with small non-coding RNA and modulating the activity of the small non-coding RNA. In a preferred embodiment of the invention the small non-coding RNA is a miRNA. In certain embodiments of the invention the antisense compounds are antisense oligonucleotides, which may comprise naturally occurring nucleosides or chemically modified nucleosides. In some embodiments, the antisense compounds comprise modified sugar moieties, modified internucleoside linkages, or modified nucleobase moieties. For convenience, the antisense compounds of the invention are herein described as being capable of modulating the activity of miRNA, but one of skill in the art, upon review of the instant disclosure, will understand that the compounds of the instant invention will also be useful for modulating other small non-coding RNA as described herein.

These antisense compounds may be further modified to impart characteristics such as, without limitation, improved pharmacokinetic or pharmacodynamic properties, binding affinity, stability, charge, localization or uptake.

As used herein, the term "small non-coding RNA" is used to encompass, without limitation, a polynucleotide molecule ranging from about 17 to about 450 nucleosides in length, which can be endogenously transcribed or produced exogenously (chemically or synthetically), but is not translated into a protein. Examples of small non-coding RNAs include, but are not limited to, primary miRNA transcripts (also known as pri-pre-miRNAs, pri-mirs, pri-miRs and pri-miRNAs, which range from around 70 nucleosides to about 450 nucleosides in length and often taking the form of a hairpin structure); pre-miRNAs (also known as pre-mirs, pre-miRs and foldback miRNA precursors, which range from around 50 nucleosides to around 110 nucleosides in length); miRNAs (also known as microRNAs, Mirs, miRs, mirs, and mature miRNAs, and generally refer either to double-stranded intermediate molecules, or to single-stranded miRNAs, which may comprise a bulged structure upon hybridization with a partially complementary target nucleic acid molecule), which range from about 19 to about 24 nucleosides in length; or mimics of pri-miRNAs, pre-miRNAs or miRNAs. For convenience, the antisense compounds of the invention are herein described as being capable of modulating the activity of miRNA, but one of skill in the art, upon review of the instant disclosure, will understand that the compounds of the instant invention will also be useful against other small non-coding RNA.

As used herein, the term "miRNA precursor" is used to encompass any longer nucleic acid sequence from which a miRNA is derived and may include, without limitation, primary RNA transcripts, pri-miRNAs, and pre-miRNAs.

In the context of the present invention, "modulation of function" means an alteration in the function or activity of the small non-coding RNA or an alteration in the function of any cellular component (including nucleic acids and proteins) with which the small non-coding RNA has an association or downstream effect (such as a downstream target regulated by a small non-coding RNA).

As used herein, the terms "target nucleic acid," "target RNA," "target RNA transcript" or "nucleic acid target" are used to encompass any nucleic acid capable of being targeted including, without limitation, small non-coding RNA. In a one embodiment, the nucleic acids are non-coding sequences including, but not limited to, miRNAs and miRNA precursors. As used herein, "miRNA nucleic acid" or "miRNA target" includes pri-miRNA, pre-miRNA, and miRNA (or mature miRNA).

In the context of the present invention, "modulation" and "modulation of expression" mean either an increase (stimulation) or a decrease (inhibition) in the level, activity, or expression of a small non-coding RNA. Small non-coding RNAs whose levels can be modulated include miRNA and miRNA precursors. More preferably, the small non-coding RNA subject to modulation is a miRNA. Inhibition is a suitable form of modulation and small non-coding RNA is a suitable target nucleic acid. Inhibition of miRNA may be detected by a change, typically an increase, in the mRNA or protein level of a miRNA target (e.g., a mRNA representing a protein-coding nucleic acid that is regulated by a miRNA).

The inhibition of small non-coding RNA level, activity or expression that results from sufficient hybridization of an antisense compound with a small non-coding RNA target nucleic acid is generally referred to as "antisense inhibition" of the small non-coding RNA.

In one embodiment, the level, activity or expression of a miRNA nucleic acid is inhibited to a degree that results in a phenotypic change, such as lowered serum cholesterol or reduced hepatic steatosis. "miRNA nucleic acid level" or "miRNA level" indicates the abundance of a miRNA in a sample, such as animal cells or tissues. miRNA level may also indicated the relative abundance of a miRNA in an experimental sample (e.g., tissue from an animal treated with an antisense compound targeted to a miRNA) as compared to a control sample (*e.g.,* tissue from an untreated animal).

### Antisense Compounds

In the context of the present invention, the term "oligomeric compound(s)" refers to polymeric structures which are capable of hybridizing to at least a region of an RNA molecule, for example, a small non-coding RNA such as a miRNA. Generally, an oligomeric compound is "antisense" to a target nucleic acid when, written in the 5' to 3' direction, it comprises the reverse complement of the corresponding region of the target nucleic acid. Such oligomeric compounds are known as "antisense compounds", which include, without limitation, oligonucleotides (*i.e.* antisense oligonucleotides), oligonucleosides, or oligonucleotide analogs.

In general, an antisense compound comprises a backbone of linked monomeric subunits where each linked monomeric subunit is directly or indirectly attached to a heterocyclic base moiety. The linkages joining the monomeric subunits, the sugar moieties or sugar surrogates, and the heterocyclic base moieties can be independently modified giving rise to a plurality of motifs for the resulting antisense compounds including hemimers, gapmers, alternating, uniformly modified, and positionally modified.

Modified antisense compounds are often preferred over native forms because of desirable properties such as, for example, enhanced cellular uptake, enhanced affinity for nucleic acid target, increased stability in the presence of nucleases and increased ability to modulate the function of a miRNA. As used herein, the term "modification" includes substitution and/or any change from a starting or natural base, nucleoside or nucleotide. Modifications to antisense compounds encompass substitutions or changes to internucleoside linkages, sugar moieties, or base moieties, such as those described below.

Antisense compounds are routinely prepared linearly but can be joined or otherwise prepared to be circular and may also include branching. Separate antisense compounds can hybridize to form double stranded compounds that can be blunt-ended or may include overhangs on one or both termini.

In one embodiment, the antisense compounds of the invention are 15 to 30 nucleosides in length, i.e. 15 to 30 linked or contiguous nucleosides. One of ordinary skill in the art will appreciate that the invention embodies antisense compounds of 15, 16, 17, 18, 19, 20, 21, 22, 23, 24, 25, 26, 27, 28, 29, or 30 nucleosides in length.

In one embodiment, the antisense compounds of the invention are 17 to 25 nucleosides in length, as exemplified herein.

In one embodiment, the antisense compounds of the invention are 19, 20, 21, 22, 23, or 24 nucleosides in length, or alternatively the antisense compounds of the invention range from 19 to 24 nucleosides in length.

In one embodiment, the antisense compounds of the invention are 21, 22, or 23 nucleosides in length, or alternatively the antisense compounds of the invention range from 21 to 23 nucleosides in length.

As used herein, the term "about" means ± 5% of the variable thereafter.

### Hybridization

"Complementary," as used herein, refers to the capacity for hybridization of two nucleobases. Conversely, a position is considered "non-complementary" when nucleobases are not capable of hybridizing. An antisense compound and a target nucleic acid are "fully complementary" to each other when each nucleobase of the antisense compound is complementary to an equal number of nucleobases at corresponding positions in the target nucleic acid.
The antisense compound and the target nucleic acid are "essentially fully complementary" to each other when the degree of precise permits stable and specific binding between the antisense compound and a target nucleic acid, so that the antisense compound inhibits the level, activity or expression of a target nucleic acid. Antisense compounds having one or two non-complementary nucleobases with respect to a miRNA may be considered essentially fully complementary. The term "sufficiently complementary" may be used in place of "essentially fully complementary.".

In the context of this invention, "hybridization" means the pairing of nucleobases of a first nucleic acid molecule with corresponding nucleobases of a second nucleic acid molecule. For example, an antisense compound hybridizes to a target nucleic acid (e.g. a miRNA nucleic acid "target" when the nucleobases of the antisense compound pair with corresponding nucleobases of the target nucleic acid. In the context of the present invention, the mechanism of pairing involves hydrogen bonding, which may be Watson-Crick, Hoogsteen or reversed Hoogsteen hydrogen bonding, between corresponding nucleobases. For example, adenine and thymine are complementary nucleobases that pair through the formation of hydrogen bonds. Hybridization can occur under varying circumstances.

It is understood in the art that the nucleobase sequence of the antisense compound need not be fully complementary to that of its target nucleic acid to be specifically hybridizable. Moreover, an antisense compound may hybridize over one or more segments such that intervening or adjacent segments are not involved in the hybridization (e.g., a bulge, a loop structure or a hairpin structure). In some embodiments there are "non-complementary" positions, also known as "mismatches", between the antisense compound and the target nucleic acid, and such non-complementary positions may be tolerated between an antisense compound and the target nucleic acid provided that the antisense compound remains specifically hybridizable to the target nucleic acid. A "non-complementary nucleobase" means a nucleobase that is unable to undergo precise base pairing with a nucleobase at a corresponding position in a target nucleic acid. As used herein, the terms "non-complementary" and "mismatch" are interchangable. Up to 3 non-complementary nucleobases are often tolerated in an antisense compound without causing a significant decrease in the ability of the antisense compound to modulate the activity, level or function of a miRNA In a preferred embodiment, the antisense compound contains 0, 1 or 2 non-complementary nucleobases with respect to a miRNA target nucleic acid. Non-complementary nucleobases may be contiguous (i.e. linked) or non-contiguous. In a more preferred embodiment, the antisense compound contains at most 1 non-complementary nucleobase with respect to a miRNA target nucleic acid.

Percent complementarity of an antisense compound with a region of a target nucleic acid can be determined routinely by those having ordinary skill in the art, and may be accomplished using BLAST programs (basic local alignment search tools) and PowerBLAST programs known in the art (Altschul et al., J. Mol. Biol., 1990, 215, 403-410; Zhang and Madden, Genome Res., 1997, 7, 649-656).

"Targeting" an antisense compound to a particular small non-coding nucleic acid molecule, including a miRNA nucleic acid, in the context of this invention, can be a multistep process. The process usually begins with the identification of a miRNA target nucleic acid whose levels, expression or function is to be modulated.

The targeting process usually also includes determination of at least one target segment within a miRNA target nucleic acid for the interaction to occur such that the desired effect, e.g., modulation of levels, expression or function of the miRNA, will result. As used herein, a "target segment means a sequence of a miRNA nucleic acid to which one or more antisense compounds are complementary. Within the context of the present invention, the term "target site" is defined as a sequence of a miRNA nucleic acid target to which an antisense compound is complementary. In some embodiments, when a single antisense compound is complementary to a target segment, a target segment and target site will be represented by the same nucleobase sequence.

Target sites and target segements may also be found in an miRNA gene from which a pri-miRNA is derived, which may be found as a solitary transcript, or it may be found within a 5' untranslated region (5'UTR), within in an intron, or within a 3' untranslated region (3'UTR) of a gene.

### Antisense compound modifications

As is known in the art, a nucleoside is a base-sugar combination. The base (or nucleobase) portion of the nucleoside is normally a heterocyclic base moiety. The two most common classes of such heterocyclic bases are purines and pyrimidines. Nucleotides are nucleosides that further include a phosphate group covalently linked to the sugar portion of the nucleoside. For those nucleosides that include a pentofuranosyl sugar, the phosphate group can be linked to the 2', 3' or 5' hydroxyl moiety of the sugar. In forming oligonucleotides, the phosphate groups covalently link adjacent nucleosides to one another to form a linear polymeric compound. Within the unmodified oligonucleotide structure, the phosphate groups are commonly referred to as forming the internucleoside linkages of the oligonucleotide. The unmodified internucleoside linkage of RNA and DNA is a 3' to 5' phosphodiester linkage.

In the context of this invention, the term "oligonucleotide" refers generally to an oligomer or polymer of ribonucleic acid (RNA) or deoxyribonucleic acid (DNA), and may be used to refer to unmodified oligonucleotides or oligonucleotide analogs. The term "unmodified oligonucleotide" refers generally to oligonucleotides composed of naturally occuring nucleobases, sugars, and covalent internucleoside linkages. The term "oligonucleotide analog" refers to oligonucleotides that have one or more non-naturally occurring nucleobases, sugars, and/or internucleoside linkages. Such non-naturally occurring oligonucleotides are often selected over naturally occurring forms because of desirable properties such as, for example, enhanced cellular uptake, enhanced affinity for other oligonucleotides or nucleic acid targets, increased stability in the presence of nucleases, or increased inhibitory activity.

### Modified Internuleoside Linkages

Specific examples of antisense compounds useful in this invention include oligonucleotides containing modified, i.e. non-naturally occurring, internucleoside linkages. Such non-naturally internucleoside linkages are often selected over naturally occurring forms because of desirable properties such as, for example, enhanced cellular uptake, enhanced affinity for other oligonucleotides or nucleic acid targets and increased stability in the presence of nucleases. Antisense compounds of the invention can have one or more modified internucleoside linkages.

One suitable phosphorus-containing modified internueleoside linkage is the phosphorothioate internucleoside linkage. In a prefered embodiment, the antisense compounds of the present invention include at least one phosphorothioate linkage or a plurality of phosphorothioate linkages. In some embodiments at least 50% of the internucleotide linkages in the antisense compound are phosphorothioate linkages. A number of other modified oligonucleotide backbones (internucleoside linkages) are known in the art and may be useful in the context of this invention. One having ordinary skill in the art can readily prepare phosphorus-containing internucleoside linkages.

### Modified Sugar Moieties

Antisense compounds of the invention may also contain one or more modified or substituted sugar moieties. The base moieties (natural, modified or a combination thereof) are maintained for hybridization with an appropriate nucleic acid target. Sugar modifications may impart nuclease stability, binding affinity or some other beneficial biological property to the antisense compounds. Representative modified sugars include sugars having substituent groups at one or more of their 2' positions and sugars having a linkage between any two other atoms in the sugar. Of the large number of sugar modifications known in the art, sugars modified at the 2' position and those which have a bridge between any 2 atoms of the sugar (such that the sugar is bicyclic) are particularly useful in this invention. Examples of sugar modifications useful in this invention include, but are not limited to compounds comprising a sugar substituent group selected from: O-, S-, or N-alkyl; or O-alkyl-O-alkyl, wherein the alkyl, alkenyl and alkynyl may be substituted or unsubstituted C₁ to C₁₀ alkyl or C₂ to C₁₀ alkenyl and alkynyl. Particularly suitable are: 2'-methoxyethoxy (also known as 2'-*O*-methoxyethyl, 2'-MOE (2'-OCH₂CH₂OCH₃), 2'-O-methyl (2'-O- CH₃), LNA™ (a bicyclic sugar moiety having a 4'-CH₂-O-2' bridge) and ENA™ (4'-(-CH₂-)₂-O-2').

One modification that imparts increased nuclease resitance and a very high binding affinity to nucleosides is the 2'-MOE side chain (Baker et al., J. Biol. Chem., 1997,272, 11944-12000). One of the immediate advantages of the 2'-MOE substitution is the improvement in binding affinity and nuclease resistance compared with similar 2'modifications such as *O*-methyl, *O*-propyl, and *O*-aminopropyl. Antisense compounds having 2'-MOE substituted sugars have been shown to be effective antisense inhibitors of gene expression for *in vivo* use (Martin, P., Helv. Chim. Acta, 1995, 78, 486-504; Altmann et al., Chimia, 1996, 50, 168-176; Altmann et al., Biochem. Soc. Trans., 1996, 24, 630-637; and Altmann et al., Nucleosides Nucleotides, 1997, 16, 917-926). Relative to DNA, the oligonucleotides having the 2'-MOE modification displayed improved RNA affinity and higher nuclease resistance. Antisense compounds having one or more 2'-MOE modifications are capable of inhibiting miRNA activity *in vitro* and *in vivo* (Esau et al., J. Biol. Chem., 2004, 279, 52361-52365; U.S. Application Publication No. 2005/0261218).

2'-Sugar substituent groups may be in the arabino (up) position or ribo (down) position. Representative U.S. patents that teach the preparation of such modified sugar structures include, but are not limited to, U.S.: 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,658,873; 5,670,633; 5,792,747; and 5,700,920, each of which is herein incorporated by reference in its entirety.

Representative substituents groups are disclosed in U.S. Patent No. 6,172,209 entitled "Capped 2'-Oxyethoxy Oligonucleotides," hereby incorporated by reference in its entirety.

Representative cyclic substituent groups are disclosed in U.S. Patent No. 6,271,358 entitled "RNA Targeted 2'-Oligomeric compounds that are Conformationally Preorganized," hereby incorporated by reference in its entirety.

Particular sugar substituent groups include O((CH₂)ₙO)ₘCH₃, O(CH₂)nOCH₃, O(CH₂)ₙNH₂, O(CH₂)ₙCH₃, O(CH₂)ₙONH₂, and O(CH₂)ₙON(CH₂)ₙCH₃))₂, where n and m are from 0 to about 10.

Representative guanidino substituent groups are disclosed in U.S. Patent 6,593,466 entitled "Functionalized Oligomers," hereby incorporated by reference in its entirety.

Representative acetamido substituent groups are disclosed in U.S. Patent 6,147,200 which is hereby incorporated by reference in its entirety.

An additional sugar modification includes a bicyclic sugar moiety, which has a 2', 4' bridge that forces the sugar ring into a locked 3'-endo conformational geometry. The bridge can be in the beta-D or alpha-L conformation. Bicyclic modifications imparts to an antisense compound greatly increased affinity for a nucleic acid target. Furthermore, nucleosides having bicyclic sugar modifications can act cooperatively with DNA and RNA in chimeric antisense compounds to enhance the affinity of a chimeric antisense compound for a nucleic acid target. Bicyclic sugar moieties can be represented by the formula 4'-(CH₂)n-X-2', where X can be, for example, O or S. What is known in the art as LNA™ is a bicyclic sugar moiety having a 4'-CH₂-O-2' bridge (i.e. X is O and n is 1). The alpha-L nucleoside has also been reported wherein the linkage is above the ring and the heterocyclic base is in the alpha rather than the beta-conformation (see U.S. Patent Application Publication No.: Application 2003/0087230). The xylo analog has also been prepared (see U.S. Patent Application Publication No.: 2003/0082807). Another bicyclic sugar moiety is ENA^{™}, which refers to a sugar moiety having a 4'-(CH₂)₂-O-2' bridge (i.e. X is O and n is 2). In general, LNA^{™} refers to the above compound when n=1, and ENA^{™} refers to the above compound when n=2 (Kaneko et al., U.S. Patent Application Publication No.: US 2002/0147332, Singh et al., Chem. Commun., 1998,4,455-456, also see U.S. Patents 6,268,490 and 6,670,461 and U.S. Patent Application Publication No.: US 2003/0207841). However the term "locked nucleic acid" can also be used in a more general sense to describe any bicyclic sugar moiety that has a "locked" conformation.

Antisense compounds incorporating LNA™ and ENA™ analogs display very high duplex thermal stabilities with complementary DNA and RNA (Tm = +3 to +10 C), stability towards 3'-exonucleolytic degradation and good solubility properties.

The synthesis and preparation of the LNA monomers adenine, cytosine, guanine, 5-methyl-cytosine, thymine and uracil, along with their oligomerization, and nucleic acid recognition properties have been described (Koshkin et al., Tetrahedron, 1998, 54, 3607-3630). LNAs and preparation thereof are also described in WO 98/39352 and WO 99/14226.

Analogs of LNA, phosphorothioate-LNA and 2'-thio-LNA (2'-S-CH2-4'), have also been prepared (Kumar et al., Bioorg. Med. Chem. Lett., 1998, 8, 2219-2222). Preparation of locked nucleoside analogs containing oligodeoxyribonucleotide duplexes as substrates for nucleic acid polymerases has also been described (Wengel et al., PCT International Application WO 98-DK393 19980914).

### Nucleobase Modifications

Antisense compounds of the invention may also contain one or more nucleobase (often referred to in the art simply as "base") modifications or substitutions which are structurally distinguishable from, yet functionally interchangeable with, naturally occurring or synthetic unmodified nucleobases. Such nucleobase modifications may impart nuclease stability, binding affinity or some other beneficial biological property to the antisense compounds. As used herein, "unmodified" or "natural" nucleobases include the purine bases adenine (A) and guanine (G), and the pyrimidine bases thymine (T), cytosine (C) and uracil (U). Modified nucleobases also referred to herein as heterocyclic base moieties include other synthetic and natural nucleobases, many examples of which such as 5-methylcytosine (5-me-C), 5-hydroxymethyl cytosine, 7-deazaguanine and 7-deazaadenine among others.

Certain nucleobase substitutions, including 5-methylcytosinse substitutions, are particularly useful for increasing the binding affinity of the antisense compounds of the invention. For example, 5-methylcytosine substitutions have been shown to increase nucleic acid duplex stability by 0.6-1.2°C (Sanghvi, Y.S., Crooke, S.T. and Lebleu, B., eds., Antisense Research and Applications, CRC Press, Boca Raton, 1993, pp. 276-278) and are presently preferred base substitutions, even more particularly when combined with 2'-O-methoxyethyl sugar modifications.

### Conjugated Oligomeric Compounds

One substitution that can be appended to the antisense compounds of the invention involves the linkage of one or more moieties or conjugates which enhance the activity, cellular distribution or cellular uptake of the resulting antisense compounds. Typical conjugates groups include cholesterol moieties and lipid moieties. Groups that enhance the pharmacokinetic properties, in the context of this invention, include groups that improve impart to the antisense compounds properties such as improved uptake, distribution, metabolism or excretion. Representative conjugate groups are disclosed in International Patent Application PCT/US92/09196, filed October 23, 1992 the entire disclosure of which is incorporated herein by reference.

Antisense compounds used in the compositions of the present invention can also be modified to have one or more stabilizing groups that are generally attached to one or both termini to enhance properties such as, for example, nuclease stability. Included in stabilizing groups are cap structures. By "cap structure" or "terminal cap moiety" is meant chemical modifications, which have been incorporated at either terminus of antisense compounds (see for example Wincott et al., WO 97/26270, incorporated by reference herein). These terminal modifications protect the antisense compounds having terminal nucleic acid molecules from exonuclease degradation, and can help in delivery and/or localization within a cell. The cap can be present at the 5'-teraninus (5'-cap) or at the 3'-terminus (3'-cap) or can be present on both termini. For double-stranded antisense compounds, the cap may be present at either or both termini of either strand. A variety of cap structures are known in the art and include, for example, inverted deoxy abasic caps.

Further 3' and 5'-stabilizing groups that can be used to cap one or both ends of an antisense compound to impart nuclease stability include those disclosed in WO 03/004602 published on January 16, 2003.

### Antisense compound motifs

It has been found that, while uniformly modified antisense compounds, such as those with substituted or unsubstituted 2'-O-alkyl substituted nucleosides, can inhibit the activity of a miRNA *in vitro* and *in vivo,* replacing one or more of the nucleosides with nucleosides containing a second, distinct modified sugar moiety (*e*.*g*.,a bicyclic sugar moiety) such that the antisense compound has a positionally modified or alternating motif may enhance its activity as compared to the activity of the uniformly modified antisense compound. In one embodiment the enhanced antisense compounds comprise a plurality of substituted or unsubstituted 2'-O-alkyl modified nucleotides (e.g. 2'-O-Methyl or 2'-methoxyethoxy) and a plurality of bicyclic modified nucleotides (e.g. LNA™ or ENA™). In a further embodiment antisense compounds which are uniformly modified with substituted or unsubstituted 2'-O-alkyl modified nucleotides are enhanced by replacing a plurality of the substituted or unsubstituted 2'-O-alkyl modified nucleotides with nucleotides containing a bicyclic sugar moiety.

As used in the present invention the term "uniform motif' is meant to include antisense compounds wherein each nucleotide bears the same type of sugar, which may be a naturally occuring sugar or a modified sugar. Further, "uniformly modified motif," "uniform modifications," and "uniformly modified" are meant to include antisense compounds wherein each nucleoside bears the same type of sugar modification. Suitable sugar modifications include, but are not limited to, 2'-O(CH₂)_{2O}CH₃ [2'-MOE], 2'-OCH₃ [2'-O-methyl], LNA^{™} and ENA^{™}. For example, an antisense compound may be uniformly modified such that each sugar modification is a 2'-MOE sugar modification. Alternatively, an antisense compound may be uniformly modified such that each sugar modification is a 2'-O-methyl sugar modification.

As used in the present invention the term "positionally modified motif' is meant to include a sequence of nucleosides having a particular sugar moiety (e.g. substitued or unsubstituted 2'-O-alkyl sugar modified nucleosides, bicyclic sugar modified nucleosides, β-D-ribonucleosides, or β-D-deoxyribonucleosides) wherein the sequence is interrupted by the introduction of two or more regions comprising from 1 to about 8 nucleosides having a different sugar moiety (e.g. if the sequence of nucleosides has a 2'-O-alkyl sugar modified nucleoside, regions may be introduced with nucleosides comprising a bicyclic sugar modification). As a consequence of the introduction of such regions, the regions having the original sugar moiety are from 1 to about 8 nucleosides in length. In other words, regions having a particular sugar moiety are separated by regions having different sugar moieties. Regions comprised of sugar-modified nucleosides may have the same sugar modification; alternatively, the modified regions may vary such that one region has a different sugar modification than another region. Positionally modified motifs are not determined by the nucleobase sequence or the location or types of internucleoside linkages.

The present invention includes antisense compounds having a positionally modified motif characterized by regions of substituted or unsubstituted 2'-O-alkyl modified sugar moieties which are separated by regions of bicyclic modified sugar moieties. Preferred substituted or unsubstituted 2'-O-alkyl modified sugar moieties include 2'-O-methyl and 2'-MOE. Preferred bicyclic sugar moieties include LNA™ and ENA™. The regions of substituted or unsubstituted 2'-O-alkyl modified sugar moieties may be 2 to 7 nucleosides in length, and the regions of bicyclic modified sugar moieties may be 1 or 2 nucleosides in length.

In one embodiment, antisense compounds of the invention are characterized by regions of two substituted or unsubstituted 2'-O-alkyl modified nucleosides separated by regions of one bicyclic modified nucleoside, such that, beginning at the 5'-terminus, the antisense compounds have a substituted or unsubstituted 2'-O-alkyl modified nucleoside at every first and second position, and a bicycle modified nucleoside at every third position. Such a motif is described by the formula 5'-(A-A-B)n(-A)nn-3', wherein A is a first sugar moiety, B is a second sugar moiety, n is 6 to 7 and nn is 0 to 2. In some embodiments, A is 2'-MOE and B is LNA™. In further embodiments, A is 2'-O-methyl and B is LNA™. In other embodiments, A is 2'-MOE and B is ENAT™. In additional embodiments, A is 2'-O-methyl and B is ENA™. In some embodiments, when such a motif would yield a bicyclic nucleoside at the 3'-terminus of the antisense compound (e.g, in an antisense compound 21 nucleosides in length), a substituted or unsubstituted 2'-O-alkyl nucleoside is incorporated in place of a bicyclic modified nucleoside. For example, if n is 7, and nn is zero, a substituted or unsubstituted 2'-O-alkyl modified nucleoside, such as 2'-MOE or 2'-O-methyl would be utilized at the 3'-terminal position in place of a bicyclic modified nucleoside, such as LNA™ or ENA™.

Positionally modified motifs having less regular patterns are also included in the present invention. For example, the majority of the substituted or unsubstituted 2'-O-alkyl modified regions may be 2 nucleosides in length, and a minority of substituted or unsubstituted 2'-O-alkyl modified regions may be 1 nucleoside in length. Likewise, the majority of the bicyclic modified regions may be 1 nucleoside in length, and a minority of the bicyclic modified regions may be 2 nucleosides in length. One non-limiting example of such an antisense compound includes a positionally modified motif as described in the preceding paragraph, having two substituted or unsubstituted 2'-O-alkyl modified regions 2 nucleosides in length, all remaining substituted or unsubstituted 2'-O-alkyl modified regions two nucleosides in length, 2 bicyclic modified region 2 nucleosides in length, and all remaining bicyclic modified regions 1 nucleoside in length.

As used in the present invention the term "alternating motif" is meant to include a contiguous sequence of alternating nucleosides, each nucleoside having a different sugar moiety (though each alternating nucleoside may have the same sugar moiety), for essentially the entire length of the antisense compound. The pattern of alternation can be described by the formula: 5'-A(B-A)n(-B)nn-3' where A and B are nucleosides differentiated by having at least different sugar moieties, nn is 0 or and n is from about 7 to about 11. This permits antisense compounds from 17 to 24 nucleosides in length. This length range is not meant to be limiting as longer and shorter antisense compounds are also amenable to the present invention. This formula also allows for even and odd lengths for alternating antisense compounds wherein the 5'- and 3'-terminal nucleosides comprise the same (odd) or different (even) sugar moieties.

Each of the A and B nucleosides has a sugar moiety selected from substituted or unsubstituted 2'-O-alkyl sugar modified nucleosides, bicyclic sugar modified nucleosides, β-D-ribonucleosides or β-D-deoxyribonucleosides (such 2'-O-alkyl sugar modified nucleosides may include 2'-MOE, and 2'-O-CH3, among others and such bicyclic sugar modified nucleosides may include LNA™ or ENA™, among others). In some embodiments, A is 2'-MOE and B is LNA™. In further embodiments, A is 2 -O-methyl and B is LNA™. In other embodiments, A is 2'-MOE and B is ENA™. In additional embodiments, A is 2'-O-methyl and B is ENA™. The alternating motif is independent from the nucleobase sequence and the internucleoside linkages. The internucleoside linkage can vary at each position or at particular selected positions or can be uniform or alternating throughout the antisense compound.

As used in the present invention the term "gapped motif' or "gapmer" is meant to include an antisense compound having an internal region (also referred to as a "gap" or "gap segment") positioned between two external regions (also referred to as "wing" or "wing segment"). The regions are differentiated by the types of sugar moieties comprising each distinct region. The types of sugar moieties that are used to differentiate the regions of a gapmer include substitued or unsubstituted 2'-O-alkyl sugar modified nucleosides, bicyclic sugar modified nucleosides, β-D-ribonucleosides or β-D-deoxyribonucleosides (such 2'-O-alkyl sugar modified nucleosides may include 2'-MOE, and 2'-O-CH3, among others and such bicyclic sugar modified nucleosides may include LNA™ or ENA™, among others). In general, each distinct region in a gapmer has uniformly modified sugar moieties.

Gapped motifs or gapmers are further defined as being either "symmetric" or "asymmetric". A gapmer wherein the nucleosides of the first wing have the same sugar modifications as the nucleosides of the second wing is termed a symmetric gapped antisense compound. Symmetric gapmers can have, for example, an internal region comprising a first type of sugar moiety, and external regions each comprising a second type of sugar moiety, wherein at least one sugar moiety is a modified sugar moiety.

Gapmers as used in the present invention include wings that independently have from 1 to 7 nucleosides. The present invention therefore includes gapmers wherein each wing independently comprises 1, 2, 3, 4, 5, 6 or 7 nucleosides. The number of nucleosides in each wing can be the same or different. In one embodiment, the internal or gap region comprises from 17 to 21 nucleosides, which is understood to include 17, 18, 19, 20, or 21 nucleosides.

As used in the present invention the term "hemimer motif" is meant to include a sequence of nucleosides that have uniform sugar moieties (identical sugars, modified or unmodified) and wherein one of the 5'-end or the 3'-end has a sequence of from 2 to 12 nucleosides that are sugar modified nucleosides that are different from the other nucleosides in the hemimer modified antisense compound. An example of a typical hemimer is an antisense compound comprising a sequence of substitued or unsubstituted 2'-O-alkyl sugar modified nucleosides, bicyclic sugar modified nucleosides, β-D-ribonucleosides or β-D-deoxyribonucleosides (such 2'-O-alkyl sugar modified nucleosides may include 2'-MOE, and 2'-O-CH3, among others and such bicyclic sugar modified nucleosides may include LNA™ or ENA™, among others) at one terminus and a sequence of nucleosides with a different sugar moiety (such as a substitued or unsubstituted 2'-O-alkyl sugar modified nucleosides, bicyclic sugar modified nucleoside) at the other terminus. One hemimer motif includes a sequence of substitued or unsubstituted 2'-O-alkyl sugar modified nucleosides at one terminus, followed or preceded by a sequence of 2 to 12 of bicyclic sugar modified nucleosides. In one embodiment, the bicyclic sugar modified nucleosides comprise less than 13 contiguous nucleosides within the antisense compound.

As used in the present invention the term "blockmer motif' is meant to include a sequence of nucleosides that have uniform sugars (identical sugars, modified or unmodified) that is internally interrupted by a block of sugar modified nucleosides that are uniformly modified and wherein the modification is different from the other nucleosides. More generally, antisense compounds having a blockmer motif comprise a sequence of substitued or unsubstituted 2'-O-alkyl sugar modified nucleosides having one internal block of from 2 to 6, or from 2 to 4, bicyclic sugar modified nucleosides. The internal block region can be at any position within the antisense compound as long as it is not at one of the termini, which would then make it a hemimer.

Antisense compounds having motifs selected from uniform, positionally modified, alternating, gapped, hemimer or blockmer may further comprise internucleoside linkage modifications or nucleobase modifications, such as those described herein.

"Chimeric antisense compounds" or "chimeras," in the context of this invention, are antisense compounds that at least 2 chemically distinct regions, each made up of at least one monomer unit, i.e., a nucleotide or nucleoside in the case of a nucleic acid based antisense compound. Accordingly, antisense compounds having a motif selected from positionally modified, gapmer, alternating, hemimer, or blockmer are considered chimeric antisense compounds.

Chimeric antisense compounds typically contain at least one region modified so as to confer increased resistance to nuclease degradation, increased cellular uptake, increased binding affinity for the target nucleic acid, and/or increased inhibitory activity.

In one aspect, the present invention is directed to antisense compounds that are designed to have enhanced properties compared to uniformly modified antisense compounds. One method to design optimized or enhanced antisense compounds involves each nucleoside of the selected sequence being scrutinized for possible enhancing modifications. One modification would be the replacement of one or more substituted or unsubstituted 2'-O-alkyl nucleosides with bicyclic sugar modified nucleosides. Such replacement can enhance the activity of the antisense compound relative to the uniformly modified antisense compound. The sequence can be further divided into regions and the nucleosides of each region evaluated for enhancing modifications that can be the result of a chimeric configuration. Consideration is also given to the 5' and 3'-termini as there are often advantageous modifications that can be made to one or more of the terminal nucleosides. The antisense compounds of the present invention may include at least one 5'-modified phosphate group on a single strand or on at least one 5'-position of a double-stranded sequence or sequences. Other modifications considered are internucleoside linkages, conjugate groups, substitute sugars or bases, substitution of one or more nucleosides with nucleoside mimetics and any other modification that can enhance the desired property of the antisense compound.

In one aspect the present invention provides antisense compounds having at least one stability enhancing nucleoside. The term "stability enhancing nucleoside" is meant to include all manner of nucleosides known to those skilled in the art to enhance stability of antisense compounds to nuclease mediated degradation (or cleavage) or spontaneous degradation (or cleavage). Examples of such stability enhancing nucleosides include, but are not limited to, bicyclic sugar modified nucleosides or substituted or unsubstituted 2'-O-alkyl sugar modified nucleosides such as those with the following modifications: 2'-methoxyethoxy (2'-O-CH₂CH₂OCH₃, Martin et al., Helv. Chim. Acta, 1995, 78, 486-504), 2'-dimethylaminooxyethoxy (O(CH₂)₂ON(CH₃)₂, 2'-dimethylaminoethoxyethoxy (2'-O-CH₂-O-CH₂-N(CH₃)₂), methoxy (-O-CH₃), aminopropoxy (-OCH₂CH₂CH₂NH₂), allyl (-CH₂-CH=CH₂), -O-allyl (-O-CH₂-CH=CH₂) and 2'-acetamido (2'-O-CH₂C(=O)NR1R1 wherein each R1 is, independently, H or C1-C1 alkyl.

Representative U.S. patents that teach the preparation of such modified sugar structures include, but are not limited to, U.S.: 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,658,873; 5,670,633; 5,792,747; and 5,700,920, each of which is herein incorporated by reference.

In one aspect the present invention provides antisense compounds having at least one stability enhancing internucleoside linkage. The term "stability enhancing internucleoside linkage" is meant to include all manner of internucleoside linkages that enhance the stability of antisense compounds to nuclease mediated degradation (or cleavage) or spontaneous degradation (or cleavage) relative to phosphodiester internucleoside linkages. An example of such stability enhancing internucleoside linkages includes, but is not limited to, phosphorothioate internucleoside linkages.

Representative U.S. patents that teach the preparation of stability enhancing internucleoside linkages include, but are not limited to, U.S.: 3,687,808; 5,286,717; 5,587,361; 5,672,697; 5,489,677; 5,663,312; 5,646,269 and 5,677,439, each of which is herein incorporated by reference.

Unless otherwise defined herein, alkyl means C₁-C₁₂, C₁-C₈, or C₁-C₆, straight or (where possible) branched chain aliphatic hydrocarbyl.

Unless otherwise defined herein, heteroalkyl (or substituted alkyl) means C₁-C₁₂, C₁-C₈, or C₁-C₆, straight or (where possible) branched chain aliphatic hydrocarbyl containing at least one, or about 1 to about 3 hetero atoms in the chain, including the terminal portion of the chain. Suitable heteroatoms include N, O and S.

Phosphate protecting groups include those described in US Patents No. US 5,760,209, US 5,614,621, US 6,051,699, US 6,020,475, US 6,326,478, US 6,169,177, US 6,121,437, US 6,465,628 each of which is expressly incorporated herein by reference in its entirety.

### Screening Antisense Compounds

Screening methods for the identification of effective modulators of small non-coding RNAs, including miRNAs, are also comprehended by the instant invention and comprise the steps of contacting a small non-coding RNA, or portion thereof, with one or more candidate modulators, and selecting for one or more candidate modulators which decrease or increase the levels, expression or alter the function of the small non-coding RNA. As described herein, the candidate modulator can be an antisense compound targeted to a miRNA, or any portion thereof. Once it is shown that the candidate modulator or modulators are capable of modulating (e.g. either decreasing or increasing) the levels, expression or altering the function of the small non-coding RNA, the modulator may then be employed in further investigative studies, or for use as a target validation, research, diagnostic, or therapeutic agent in accordance with the present invention. In one embodiment, the candidate modulator is screened for its ability to modulate the function of a specific miRNA.

### Antisense Compound Synthesis

Antisense compounds and phosphoramidites are made by methods well known to those skilled in the art. Oligomerization of modified and unmodified nucleosides is performed according to literature procedures for DNA like compounds (Protocols for Oligonucleotides and Analogs, Ed. Agrawal (1993), Humana Press) and/or RNA like compounds (Scaringe, Methods (2001), 23, 206-217. Gait et al., Applications of Chemically synthesized RNA in RNA:Protein Interactions, Ed. Smith (1998), 1-36. Gallo et al., Tetrahedron (2001), 57, 5707-5713) synthesis as appropriate. Alternatively, antisense may be purchased from various oligonucleotide synthesis companies such as, for example, Dharmacon Research Inc., (Lafayette, CO).

Irrespective of the particular protocol used, the antisense compounds used in accordance with this invention may be conveniently and routinely made through the well-known technique of solid phase synthesis. Equipment for such synthesis is sold by several vendors including, for example, Applied Biosystems (Foster City, CA). Any other means for such synthesis known in the art mav additionally or alternatively be employed (including solution phase synthesis).

Methods of isolation and analysis of oligonucleotides are well known in the art. A 96-well plate format is particularly useful for the synthesis, isolation and analysis of oligonucleotides for small scale applications.

### Diagnostics, Drug Discovery and Therapeutics

The antisense compounds and compositions of the present invention can additionally be utilized for research, drug discovery, and therapeutics.

For use in research, antisense compounds of the present invention are used to interfere with the normal function of the nucleic acid molecules to which they are targeted. Expression patterns within cells or tissues treated with one or more antisense compounds or compositions of the invention are compared to control cells or tissues not treated with the compounds or compositions and the patterns produced are analyzed for differential levels of nucleic acid expression as they pertain, for example, to disease association, signaling pathway, cellular localization, expression level, size, structure or function of the genes examined. These analyses can be performed on stimulated or unstimulated cells and in the presence or absence of other compounds that affect expression patterns.

For use in drug discovery, antisense compounds of the present invention are used to elucidate relationships that exist between small non-coding RNAs, genes or proteins and a disease state, phenotype, or condition. These methods include detecting or modulating a target comprising contacting a sample, tissue, cell, or organism with the antisense compounds and compositions of the present invention, measuring the levels of the target and/or the levels of downstream gene products including mRNA or proteins encoded thereby, a related phenotypic or chemical endpoint at some time after treatment, and optionally comparing the measured value to an untreated sample, a positive control or a negative control. These methods can also be performed in parallel or in combination with other experiments to determine the function of unknown genes for the process of target validation or to determine the validity of a particular gene product as a target for treatment or prevention of a disease.

The specificity and sensitivity of antisense compounds and compositions can also be harnessed by those of skill in the art for therapeutic uses. Antisense compounds have been employed as therapeutic moieties in the treatment of disease states in animals, including humans. Antisense oligonucleotide drugs, including ribozymes, have been safely and effectively administered to humans and numerous clinical trials are presently underway. It is thus established that antisense compounds can be useful therapeutic modalities that can be configured to be useful in treatment regimes for the treatment of cells, tissues and animals, especially humans.

For therapeutics, an animal, preferably a human, suspected of having a disease or disorder presenting conditions that can be treated, ameliorated, or improved by modulating the expression or a selection sman non-coding target nucleic acid is treated by administering the compounds and compositions of the present invention. Antisense compounds of the instant invention are expected to exhibit greater stability and activity in animals than unmodified oligonucleotides and thus may be preferred for therapeutic applications. For example, in one non-limiting embodiment, the methods comprise the step of administering to or contacting the animal, an effective amount of a modulator or mimic to treat, ameliorate or improve the conditions associated with the disease or disorder. The compounds of the present invention effectively modulate the activity or function of the small non-coding RNA target or inhibit the expression or levels of the small non-coding RNA target. In preferred embodiments, the small non-coding RNA target is a miRNAIn another embodiment, the present invention provides for the use of a compound of the invention in the manufacture of a medicament for the treatment of any and all conditions associated with a miRNA target nucleic acid.

The reduction of small non-coding RNA may be measured in serum, adipose tissue, liver or any other body fluid, tissue or organ of the animal known to contain the small non-coding RNA or its precursor. Further, the cells contained within the fluids, tissues or organs being analyzed contain a nucleic acid molecule of a downstream target regulated or modulated by the small non-coding RNA target itself

### Compositions and Methods for Formulating Pharmaceutical Compositions

The present invention also include pharmaceutical compositions and formulations that include the antisense compounds and compositions of the invention. Compositions and methods for the formulation of pharmaceutical compositions are dependent upon a number of criteria, including, but not limited to, route of administration, extent of disease, or dose to be administered. Such considerations are well understood by those skilled in the art.

The antisense compounds and compositions of the invention can be utilized in pharmaceutical compositions by adding an effective amount of the compound or composition to a suitable pharmaceutically acceptable diluent or carrier. Use of the antisense compounds and methods of the invention may also be useful prophylactically.

The antisense compounds and compositions of the invention encompass any pharmaceutically acceptable salts, esters, or salts of such esters, or any other compound which, upon administration to an animal, including a human, is capable of providing (directly or indirectly) the biologically active metabolite or residue thereof. Accordingly, for example, the disclosure is also drawn to prodrugs and pharmaceutically acceptable salts of the antisense compounds of the invention, pharmaceutically acceptable salts of such prodrugs, and other bioequivalents.

The term prodrug indicates a therapeutic agent that is prepared in an inactive form that is converted to an active form (i.e., drug) within the body or cells thereof by the action of endogenous enzymes or other chemicals and/or conditions. Such preparation can include the incorporation of additional nucleosides at one or both ends of an antisense compound which are cleaved by nucleases present in an animal cell, such that the active antisense compound is formed.

The term "pharmaceutically acceptable salts" refers to physiologically and pharmaceutically acceptable salts of the antisense compounds and compositions of the invention: i.e., salts that retain the desired biological activity of the parent antisense compound and do not impart undesired toxicological effects thereto. Suitable examples include, but are not limited to, sodium and postassium salts.

In some embodiments, an antisense compound can be administered to a subject via an oral route of administration. The subject may be a mammal, such as a mouse, a rat, a dog, a guinea pig, or a non-human primate. In some embodiments, the subject may be a human or a human patient. In certain embodiments, the subject may be in need of modulation of the level or expression of one or more miRNAs. In some embodiments, compositions for administration to a subject will comprise modified oligonucleotides having one or more modifications, as described herein. A suitable method of administration is parenteral administration, which includes, for example, intravenous administration, subcutaneous administration, and intraperitoneal administration.

### Cell culture and oligonucleotide treatment

The effects of antisense compounds on level, activity or expression of small non-coding RNAs, or their protein-coding RNA targets, can be tested in any of a variety of cell types provided that the target nucleic acid is present at measurable levels. This can be readily determined by methods routine in the art, for example Northern blot analysis, ribonuclease protection assays, or real-time PCR. Cell types used for such analyses are available from commerical vendors (e.g. American Type Culture Collection, Manassus, VA; Zen-Bio, Inc., Research Triangle Park, NC; Clonetics Corporation, Walkersville, MD) and cells are cultured according to the vendor's instructions using commercially available reagents (e.g. Invitrogen Life Technologies, Carlsbad, CA). Illustrative cell types include, but are not limited to: T-24 cells, A549 cells, normal human mammary epithelial cells (HMECs), MCF7 cells, T47D cells, BJ cells, B16-F10 cells, human vascular endothelial cells (HUVECs), human neonatal dermal fibroblast (NHDF) cells, human embryonic keratinocytes (HEK), 293T cells , HepG2 , human preadipocytes, human differentiated adipocytes (preapidocytes differentiated according to methods known in the art), NT2 cells (also known as NTERA-2 cl.D1), and HeLa cells.

### Treatment with antisense compounds

In general, when cells reach approximately 60-80% confluency, they are treated with antisense compounds of the invention.

One reagent commonly used to introduce antisense compounds into cultured cells includes the cationic lipid transfection reagent LIPOFECTIN® (Invitrogen, Carlsbad, CA). Antisense compounds are mixed with LIPOFECTIN® in OPTI-MEM® 1 (Invitrogen, Carlsbad, CA) to achieve the desired final concentration of antisense compound and a LIPOFECTIN® concentration that typically ranges 2 to 12 µg/mL per 100 nM antisense compound.

Another reagent used to introduce antisense compounds into cultured cells includes LIPOFECTAMINE® (Invitrogen, Carlsbad, CA). Antisense compound is mixed with LIPOFECTAMINE® in OPTI-MEM® 1 reduced serum medium (Invitrogen, Carlsbad, CA) to achieve the desired concentration of antisense compound and a LIPOFECTAMINE® concentration that typically ranges 2 to 12 µg/mL per 100 nM antisense compound.

Cells are treated with antisense compounds by routine methods well known to those skilled in the art. Cells are typically harvested 16-24 hours after antisense compound treatment, at which time RNA or protein levels of target nucleic acids are measured by methods known in the art and described herein. When the target nucleic acid is a miRNA, the RNA or protein level of a protein-coding RNA regulated by a miRNA may be measured to evaluate the effects of antisense compounds targeted to a miRNA. In general, when treatments are performed in multiple replicates, the data are presented as the average of the replicate treatments.

The concentration of antisense used varies from cell line to cell line. Methods to determine the optimal antisense concentration for a particular cell line are well known in the art. Antisense compounds are typically used at concentrations ranging from 1 nM to 300 nM.

### RNA Isolation

RNA analysis can be performed on total cellular RNA or poly(A)+ mRNA. Methods of RNA isolation are well known in the art. RNA is prepared using methods well known in the art, for example, using the TRIZOL® Reagent (Invitrogen, Carlsbad, CA) according to the manufacturer's recommended protocols.

### Analysis of antisense inhibition of target levels or expression

Modulation of the levels miRNAs or of protein-coding RNAs regulated by miRNAs can be assayed in a variety of ways known in the art. For example, nucleic acid levels can be quantitated by, e.g., Northern blot analysis, competitive polymerase chain reaction (PCR), or quantitative real-time PCR. Northern blot analysis is also routine in the art. Quantitative real-time PCR can be conveniently accomplished using the commercially available ABI PRISM® 7600, 7700, or 7900 Sequence Detection System, available from PE-Applied Biosystems, Foster City, CA and used according to manufacturer's instructions.

Additional examples of methods of gene expression analysis known in the art include DNA arrays or microarrays (Brazma and Vilo, FEBS Lett., 2000, 480, 17-24; Celis, et al., FEBS Lett., 2000, 480, 2-16), SAGE (serial analysis of gene expression)(Madden, et al., Drug Discov. Today, 2000, 5, 415-425), READS (restriction enzyme amplification of digested cDNAs) (Prashar and Weissman, Methods Enzymol., 1999, 303, 258-72), TOGA (total gene expression analysis) (Sutcliffe, et al., Proc. Natl. Acad. Sci. U. S. A., 2000, 97, 1976-81), protein arrays and proteomics (Celis, et al., FEBS Lett., 2000, 480, 2-16; Jungblut, et al., Electrophoresis, 1999, 20, 2100-10), expressed sequence tag (EST) sequencing (Celis, et al., FEBS Lett., 2000, 480, 2-16; Larsson, et al., J. Biotechnol., 2000, 80, 143-57), subtractive RNA fingerprinting (SuRF) (Fuchs, et al., Anal. Biochem., 2000, 286, 91-98; Larson, et al., Cytometry, 2000, 41, 203-208), subtractive cloning, differential display (DD) (Jurecic and Belmont, Curr. Opin. Microbiol., 2000, 3,316-21), comparative genomic hybridization (Carulli, et al., J. Cell Biochem. Suppl., 1998, 31, 286-96), FISH (fluorescent in situ hybridization) techniques (Going and Gusterson, Eur. J. Cancer, 1999, 35, 1895-904), and mass spectrometry methods (To, Comb. Chem. High Throughput Screen, 2000, 3, 235-41).

### Quantitative Real-Time PCR Analysis of Target RNA Levels

Quantitation of RNA levels is accomplished by quantitative real-time PCR using the ABI PRISM® 7600, 7700, or 7900 Sequence Detection System (PE-Applied Biosystems, Foster City, CA) according to manufacturer's instructions. Methods of quantitative real-time PCR are well known in the art.

Prior to real-time PCR, the isolated RNA is subjected to a reverse transcriptase (RT) reaction, which produces complementary DNA (cDNA) that is then used as the substrate for the real-time PCR amplification. The RT and real-time PCR reactions are performed sequentially in the same sample well. RT and real-time PCR reagents are obtained from Invitrogen (Carlsbad, CA). RT, real-time-PCR reactions are carried out by methods well known to those skilled in the art.

Gene (or RNA) target quantities obtained by real time PCR are normalized using either the expression level of a gene whose expression is constant, such as GAPDH, or by quantifying total RNA using RIBOGREEN® (Molecular Probes, Inc. Eugene, OR). GAPDH expression is quantified by real time PCR, by being run simultaneously with the target, multiplexing, or separately.

Total RNA is quantified using RIBOGREEN® RNA quantification reagent (Molecular Probes, Inc. Eugene, OR), according to the manufacturer's recommended protocols. Methods of RMA quantification by RIBOGREEN® are taught in Jones, L.J., et al, (Analytical Biochemistry, 1998, 265, 368-374). A CYTOFLUOR® 4000 instrument (PE Applied Biosystems) is used to measure RIBOGREEN® fluorescence.

Probes and primers are designed to hybridize to the target sequence, which includes a protein-coding RNA that is regulated by a miRNA. Methods for designing real-time PCR probes and primers are well known in the art, and may include the use of software such as PRIMER EXPRESS® Software (Applied Biosystems, Foster City, CA).

### Northern blot analysis of miRNA levels

Northern blot analysis is performed according to routine procedures known in the art. Higher percentage acrylamide gels, for example, 10 to 15% acrylamide urea gels, are generally used to resolve miRNA. Fifteen to twenty micrograms of total RNA is fractionated by electrophoresis. RNA is transferred from the gel to HYBOND™-N+ nylon membranes (Amersham Pharmacia Biotech, Piscataway, NJ) by electroblotting in an Xcell SURELOCK™ Minicell (Invitrogen, Carlsbad, CA). Membranes are fixed by UV cross-linking using a STRATALINKER® UV Crosslinker 2400 (Stratagene, Inc, La Jolla, CA) and then probed using RAPID-HYB™ buffer solution (Amersham) using manufacturer's recommendations for oligonucleotide probes.

A target specific DNA oligonucleotide probe with the sequence is used to detect the RNA of interest. Probes used to detect miRNAs are synthesized by commercial vendors such as IDT (Coralville, IA). The probe is 5' end-labeled with T4 polynucleotide kinase with (γ-³²P) ATP (Promega, Madison, WI). To normalize for variations in loading and transfer efficiency membranes are stripped and re-probed for an RNA whose level is constant, such as GAPDH. For higher percentage acrylamide gels used to resolve miRNA, U6 RNA is used to normalize for variations in loading and transfer efficiency. Hybridized membranes are visualized and quantitated using a STORM® 860 PHOSPHORIMAGER® System and IMAGEQUANT® Software V3.3 (Molecular Dynamics, Sunnyvale, CA).

### Analysis of Protein Levels

Protein levels of a downstream target modulated or regulated by miRNA can be evaluated or quantitated in a variety of ways well known in the art, such as immunoprecipitation, Western blot analysis (immunoblotting), enzyme-linked immunosorbent assay (ELISA), quantitative protein assays, protein activity assays (for example, caspase activity assays), immunohistochemistry, immunocytochemistry or fluorescence-activated cell sorting (FACS). Antibodies directed to a target can be identified and obtained from a variety of sources, such as the MSRS catalog of antibodies (Aeric Corporation, Birmingham, MI), or can be prepared via conventional monoclonal or polyclonal antibody generation methods well known in the art.

### Phenotypic assays

Once modulators are designed or identified by the methods disclosed herein, the antisense compounds are further investigated in one or more phenotypic assays, each having measurable endpoints predictive or suggestive of efficacy in the treatment, amelioration or improvement of physiologic conditions associated with a particular disease state or condition.

Phenotypic assays, kits and reagents for their use are well known to those skilled in the art and are herein used to investigate the role and/or association of a target in health and disease. Representative phenotypic assays include cell cycle assays, apoptosis assays, angiogenesis assays (e.g. endothelial tube formation assays, angiogenic gene expression assays, matrix metalloprotease activity assays), adipocyte assays (e.g. insulin signaling assays, adipocyte differentiation assays), inflammation assays (e.g. cytokine signaling assays, dendritic cell cytokine production assays); examples of such assays are readily found in the art (*e.g.,* U.S. Application Publication No. 2005/0261218, which is hereby incorporated by reference in its entirety). Additional phenotypic assays include those that evaluate differentiation and dedifferentiation of stem cells, for example, adult stem cells and embryonic stem cells; protocols for these assays are also well known in the art (e.g. Turksen, Embryonic Stem Cells: Methods and Protocols, 2001, Humana Press; Totowa, NJ; Klug, Hematopoietic Stem Cell Protocols, 2001, Humana Press, Totowa, NJ; Zigova, Neural Stem Cells: Methods and Protocols, 2002, Humana Press, Totowa, NJ).

Various modifications of the invention, in addition to those described herein, will be apparent to those skilled in the art from the foregoing description. Such modifications are also intended to fall within the scope of the appended claims. Each reference (including, but not limited to, journal articles, U.S. and non-U.S. patents, patent application publications, international patent application publications, GENBANK® accession numbers, and the like) cited in the present application is specifically incorporated herein by reference in its entirety.

In order that the invention disclosed herein may be more efficiently understood, examples are provided below. It should be understood that these examples are for illustrative purposes only and are not to be construed as limiting the invention in any manner. Throughout these examples, molecular cloning reactions, and other standard recombinant DNA techniques, were carried out according to routine methods, such as those described in Maniatis et al., Molecular Cloning - A Laboratory Manual, 2nd ed., Cold Spring Harbor Press (1989), using commercially available reagents, except where otherwise noted.

### Examples

The following non-limiting examples are useful in describing the current discovery, and are in no way meant to limit the invention. Those of ordinary skill in the art will readily adopt the underlying principles of this discovery to design various compounds without departing from the spirit of the current invention.

### Example 1: Uniformly Modified Antisense Compounds_{.}

As defined herein, uniformly modified antisense compounds are those in which each nucleoside bears the same sugar modification. In one embodiment, each sugar modification comprises a 2'-MOE sugar modification. In a further embodiment, each sugar modification comprises a 2'-O-methyl modification. Such uniformly modified antisense compounds may further comprise modified internucleoside linkages, such as phosphorothioate linkages, and/or modified nucleobases, such as 5-methylcytosine. For example antisense compounds are uniformly modified with 2'-MOE sugar moieties, and each internucleoside linkage is a phosphorothioate linkage.

### Example 2: Positionally Modified and Alternating Antisense Compounds.

In this example, the antisense compounds comprise sugar modifications applied to specific positions in the antisense compound, and as such are considered positional modifications. Also illustrated in this example are antisense compounds having an alternating motif

In a further embodiment of a positionally modified or alternating motif antisense compound, the antisense compounds further comprise an internucleoside linkage modification such as a phosphorothioate linkage.

In yet a further embodiment of a positionally modified or alternating motif antisense compound, the antisense compound comprises a nucleobase modification such as 5-methylcytosine in place of unmodified cytosine.

In each of the following positionally modified or alternating motifs, A represents a nucleoside having a first sugar moiety and B represents a nucleoside having a second sugar moiety. Where present, C represents a nucleotide having a third sugar moiety. In one embodiment, A is a nucleoside with an unmodified sugar moiety and B is a nucleoside with a modified sugar moiety. In a further embodiment, A is a modified sugar moiety and B is an unmodified sugar moiety. In yet another embodiment, both A and B are nucleosides with distinct modified sugar moieties. In preferred embodiments, A is 2'-MOE and B is LNA. In further preferred embodiments, A is 2 -O-methyl and B is LNA. In another embodiment, A is 2'-MOE and B is ENA. In a further embodiment, A is 2'-O-methyl and B is ENA.

Examples of positionally modified antisense compounds include, but are not limited to, an antisense compound having the motif (5''ABABABABABABABABABABAA3'); an antisense compound having the motif (5'BAAABBAAAABBABBBBBBA3'); and an antisense compound having the motif (5'-AABBABAAABBBBAAAABBBBB-3').

An additional example of a positionally motif includes (A-A-B)n(-A)nn, where n ranges from 6 to 8 and nn ranges from 0 to 2. In one embodiment, n is 7 and nn is 2. In another embodiment, n is 7 and nn is 1. In a further embodiment, n is 7 and nn is zero

A further example of a positionally modified antisense compound includes an antisense compound comprising clusters of three distinct sugar moieties, one of which is unmodified and two of which are modified. For example, such an antisense compound may have the motif (5'-ABBACCCBBAABCCCCBBBCCAA-3'); In one preferred embodiment of this motif, A represents a nucleoside having a first sugar modification, B represents a nucleoside having a second sugar modification, and C represents a nucleoside without a sugar modification. Alternatively, a 23 nucleoside antisense compound may have the motif 5'-(A-A-B)nn-(C-C-B-A-A-B)nn-(-A-A-B)nn(A-A)n-3', where n is 1 and nn is 2, "A" represents the first modification, "B" represents the second modification, and "C" represents an unmodified sugar moiety.

An example of an alternating motif antisense compound includes an antisense compound having the motif 5'-(A-B)n-(A)nn-3', where n is from 10 to 11, and nn is 0 or 1, and A and B are as above. In one embodiment, n is 11 and nn is 1. In another embodiment, n is 11 and nn is zero.

### Example 3: Examples of chemically modified motifs

Shown in Table 1 are antisense compounds containing motifs illustrative of those that can be applied to enhance the ability of antisense compounds to inhibit miRNA.

Motifs may be described by a formula, such as (A-B)₁₁-(A)₁, where A is a nucleoside having first sugar moiety and B is a nucleoside having a second sugar moiety, and the subscripted numbers indicate the number of repeating regions comprised of each nucleoside or block of nucleosides. An alternative notation is, for example "A₅-B₁-A₅-B₁-A₄-B₁-A₆", which indicates that five nucleosides having an "A" sugar moiety are followed by one nucleoside having a "B" sugar moiety, and so forth. Some motifs use a combination of the aforementioned notations. Where present, "C" indicates a nucleoside having a third sugar moiety.

"Sugar" denotes the type of sugar moiety in the nucleosides of the example compounds and is abbreviated, for instance as 2'-MOE, 2'-O-methyl, 2'-deoxy, or LNA™ "Backbone" is abbreviated as PS for phosphorothioate and PO for phosphodiester. "MIXED" backbones are those where the first two and last three internucleoside linkages are phosphorothioate, and the remaining internucleoside linkages are phosphodiester.

As described herein, an antisense compound having any of the aforementioned motifs can further comprise modified nucleobases, such as 5'-methylcytosines.

**Table 1: Antisense compound motifs**

| **Motif 5' TO 3'** | **Sugar** | **Backbone** |
|---|---|---|
| Uniform | RNA | PO |
| Uniform | RNA | PS |
| Uniform | 2'-MOE | PS |
| Uniform | 2'-MOE | PO |
| Uniform | 2'-MOE | MIXED |
| Uniform | 2'-O-Methyl | PS |
| Uniform | 2'-O-Methyl | PO |
| Uniform | 2'-O-Methyl | MIXED |
| Positionally modified A₅-B₁-A₅-B₁-A₄-B₁-A₆ OR (A-A-A-A-A-B)₂-A₄-B-A₆ | A = 2'-MOE B = LNA | PS |
| Positionally modified A₅-B₁-A₂-B₁-A₂-B₁-A₂-B₁-A₁-B₁-A₃-B₁-A₂ OR A₅(-B-A-A)₃-A-B-A₃-B-A₂ | A = 2'-MOE B = LNA | PS |
| Positionally modified (A-A-B)₇(-A)₂ | A = 2'-MOE B = LNA | PS |
| Positionally modified (A-A-B)₇(-A)₂ | A = 2'-MOE B = LNA | PO |
| Positionally modified A₃-B₃-A₂-B₃-A₂-B₃-A₇ OR A₃(-B-B-B-A-A)₂-B₃-A₇ | A = 2'MOE B = 2'-Deoxy | PS |
| Alternating (A-B)₁₁-(A)₁ | A = 2'-MOE B = 2'-doxy | PS |
| Positionally modified A₃-B₃-A₂-B₃-A₂-B₃-A₂-B₃-A₂ OR A₃(-B-B-B-A-A)₄ | A = 2'-MOE B = 2'-Deoxy | PS |
| Positionally modified (A-A-A-B)₅-A₃ | A = 2'-MOE B = LNA | PS |
| Positionally modified A₃-B₂-A₂-B₃-A₂-B₂-A₈ | A = 2'-MOE B = Deoxy | PS |
| Positionally modified A₅-B₂-A₂-B₃-A₂-B₃-A₅ | A = 2'-MOE B = Deoxy | PS |
| Positionally modified A₆-B₁-A₉-B₂-A₃-B₁-A₁ | A= 2'-MOE B = 2'-O-Methyl | MIXED |
| Positionally modified (A-A-B)₇-A-A | A = 2'-MOE B = LNA | PS |
| Positionally modified (A-A-B)₇-A-A | A = 2'-O-Methyl B = LNA | PS |
| Positionally modified (A-A-B)₂-(C-C-B-A-A-B)₂-(A-A-B)ₜ(-A-A)₁ | A = 2'-MOE B = LNA C = 2'-deoxy | PS |

### Example 4: Modulation Activities for Enhanced Antisense Compounds.

In these following examples, an antisense compound directed towards miR-21 is illustrated; however, the modifications in these are not limited to only those antisense compounds that modulate miR-21.

### Dual-Luciferase Reporter Assay.

A miR-21 luciferase sensor construct was engineered using pGL3-MCS2 (Promega). Day1: Hela cells (ATCC) were seeded in T-170 flasks (BD Falcon) at 3.5 *10⁶ cells/flask. Hela cells were grown in Dulbecco's Modified Eagle Medium with High Glucose (Invitrogen). Day 2: Each flask of Hela cells was transfected with 10ug luciferase sensor construct engineered to contain the full 22 nucleobase sequence complementary to the mature miR-21 sequence. Each flask was also transfected with 0.5ug of a phRL sensor plasmid (Promega) expressing Renilla to be used in normalization. Hela cells were transfected using 20ul Lipofectamine 2000/flask (Invitrogen). After 4 hours of transfection, cells were washed with PBS and trypsinized. Hela cells were plated at 40k/well in 24 well plates (BD Falcon) and left overnight. Day 3: Hela cells were transfected with antisense compounds (also referred to as "ASO" for antisense oligonucleotides) using Lipofectin (Invitrogen) at 2.5ul Lipofectin/100nM ASO/ml Opti-MEM I Reduced Serum Medium (Invitrogen) for 4 hours. After ASO transfection, Hela cells were refed with Dulbecco's Modified Eagle Medium with High Glucose (Invitrogen). Day 4: Hela cells are passively lysed and luciferase activity measured using the Dual-Luciferase Reporter Assay System (Promega).

### Effects of 2'-sugar substitutions on antisense compound activity

In a one assay, the effect of sugar modifications on activity of an antisense compound targeted to a miRNA was determined. Hela cells were treated with anti-miR-21 antisense compounds with uniform 2'-MOE or 2'-O-methyl sugar modifications. Also tested was a positionally modified antisense compound, having 2'-MOE sugar modifications separated by a single LNA™ modification at every third position [i.e. (A-A-B)n(A-A)nn, where A is 2'-MOE, B is LNA™, n is 7 and nn is 1]. Each of these compounds had phosphorothioate linkages throughout the compound. Among these antisense compounds with phosphorothioate (PS) backbones, the 2'-MOE uniformly modified and the 2'-O-methyl uniformly modified antisense compounds exhibited approximately equal antisense inhibition of miR-21. The introduction of LNA™ sugar modifications into a 2'-MOE uniformly modified background enhanced the ability of the antisense compound to inhibit miR-21 activity. Thus, it was demonstrated that the introduction of a bicyclic sugar moiety into an otherwise uniformly modified background enhanced the ability of an antisense compound to inhibit a miRNA.

### Effect of phosphodiester backbone on anti-miRNA activity

Also evaluated in the luciferase sensor assay were uniform 2'-MOE and uniform 2'-O-methyl antisense compounds having unmodified, phosphodiester (PO) internucleoside linkages. The 2'-MOE uniformly modified, PO compound exhibited greater anti-miR-21 activity compared to the PS version of the same compound. However, changing the backbone of the 2'-O-methyl uniformly modified antisense compound to PO yielded no increase in anti-miR-21 activity.

### Example 5: Antisense inhibition miRNA activity in vivo using enhanced antisense compounds

The antisense compounds of the invention modulate the activity or function of the small non-coding RNAs to which they are targeted. In this example, antisense compounds targeted to miR-122a are illustrated; however, the modifications in the antisense compounds of the invention are not limited to those antisense compounds that modulate miR-122a.

Male C57BL/6 mice were obtained from The Jackson Laboratory. Mice were treated with antisense compounds targeting miR-122a, or received saline as a control treatment.

One of the antisense compounds tested included a 2'-MOE uniformly modified antisense compound fully complementary to miR-122a. Also tested *in vivo* was a positionally modified antisense compound fully complementary to miR-122a having the motif (A-A-B)₇(-A-A)₁ where A is 2'-MOE and B is LNA.

Mice were administered 25 mg/kg doses of antisense compound intraperitoneally, for a total of 6 doses. Following the end of the treatment period, RNA was isolated from liver and the levels of a miR-122a target mRNA, ALDOA, were measured using Taqman real-time PCR. Relative to saline-treated animals, treatment with the 2'-MOE uniformly modified compound resulted in ALDO A mRNA levels approximately 4 times those in saline-treated animals. Treatment with the positionally modified compound resulted in ALDO A mRNA levels approximately 5 times those observed in saline-treated animals. Thus, incorporation of a bicyclic sugar moiety into an otherwise uniformly modified background enhanced the ability of an antisense compound to inhibit miR-122a activity *in vivo.*

Plasma levels of total cholesterol were also monitored using methods known in the art (for example, via Olympus AU400e automated clinical chemistry analyzer, Melville, NY). Reductions in total cholesterol were observed in mice treated with either the 2'-MOE uniformly modified antisense compound or the positionally modified antisense compound having a plurality of 2'-MOE modified nucleosides and a plurality of LNA™ modified nucleosides.

Additional analyses that are performed in such in vivo studies included histological analysis of liver sections, to evaluate changes in morphology. Histological analysis of liver is carried out via routine procedures known in the art. Briefly, liver is fixed in 10% buffered formalin and embedded in paraffin wax. 4-mm sections are cut and mounted on glass slides. After dehydration, the sections are stained with hematoxylin and eosin. Morphological analysis may also include evaluation of hepatic steatosis, using oil Red O staining procedures known in the art.

Various modifications of the invention, in addition to those described herein, will be apparent to those skilled in the art from the foregoing description. Such modifications are also intended to fall within the scope of the appended claims. Each reference (including, but not limited to, journal articles, U.S. and non-U.S. patents, patent application publications, international patent application publications, GENBANK® accession numbers, and the like) cited in the present application is specifically incorporated herein by reference in its entirety.

The following embodiments are also disclosed herein:
1. An antisense compound comprising a plurality of nucleosides with substituted or unsubstituted 2'-O-alkyl modified nucleosides and a plurality of nucleosides with bicyclic sugar modified nucleosides.
2. The antisense compound of embodiment 1 wherein each of said substituted or unsubstituted 2'-O-alkyl modified nucleosides have the same sugar modification and each of said bicyclic sugar modified nucleosides have the same bicyclic modification.
3. The antisense compound of embodiment 1 or embodiment 2 wherein the 2'-substituent group each of said substituted or unsubstituted 2'-O-alkyl modified nucleosides is, independently, -O-(CH₂)ⱼ-CH₃, -O-(CH₂)₂-O-CH₃, -O(CH₂)₂-S-CH₃, O-(CH₂)₂-O-N(Rₘ)(Rₙ) or O-CH₂-C(=O)-N(Rₘ)(Rₙ), where j is 0, 1 or 2 and each Rₘ and Rₙ is, independently, H, an amino protecting group or substituted or unsubstituted C1-C10 alkyl.
4. The antisense compound of embodiment 1 or 2 wherein the wherein the bicyclic sugar of each bicyclic sugar modified nucleoside comprises a 2'-O-CH₂-4', or a 2'-O-(CH₂)₂-4' bridge.
5. The antisense compound of embodiment 1 wherein said antisense compound comprises linked substituted or unsubstituted 2'-O-alkyl modified nucleosides having at least two internal regions of bicyclic sugar modified nucleosides wherein each internal region comprises from 1 to 4 bicyclic sugar modified nucleosides.
6. The antisense compound of embodiment 5 comprising from 3 to about 7 internal regions of bicyclic sugar modified nucleosides.
7. The antisense compound of embodiment 6 wherein each region of bicyclic sugar modified nucleosides is flanked on each side by from 1 to about 8 substituted or unsubstituted 2'-O-alkyl modified nucleosides.
8. The antisense compound of embodiment 7 having one of the formulas: A₅-B₁-A₅-B₁-A₄-B₁-A₆, (A-A-B)₇(-A)₂, (A-A-A-B)₅-A₃, A₅-B₁-A₂-B₁-A₂-B₁-A₂-B₁-A₁-B₁-A₃-B₁-A₂, A₃-B₃-A₂-B₃-A₂-B₃-A₇, A₃-B₃-A₂-B₃-A₂-B₃-A₂-B₃-A₂, A₃-B₂-A₂-B₃-A₂-B₂-A₈, A₅-B₂-A₂-B₃-A₂-B₃-A₅,
   wherein A is a substituted or unsubstituted 2'-O-alkyl modified nucleosides, B is a bicyclic sugar modified nucleoside, and each subscript number represents the number of repeats of the preceding nucleoside or block of nucleosides.
9. The antisense compound of embodiment 8 wherein each 2'-substituent group of said substituted or unsubstituted 2'-O-alkyl modified nucleosides is -O-(CH₂)₂-O-CH₃ and each bicyclic modified nucleoside comprises a 2'-O-CH₂-4' bridge.
10. The antisense compound of any of embodiments 1 to 9 comprising from about 15 to about 30 linked nucleosides.
11. The antisense compound of any of embodiments 1 to 10 wherein each internucleoside linking group is, independently, a phosphodiester or a phosphorothioate.
12. The antisense compound of embodiment 11 further comprising a plurality of phosphorothioate internucleoside linkages.
13. The antisense compound of any of embodiments 1 to 12 further comprising one or more regions of from 1 to 4 differentially modified nucleosides wherein said differentially modified nucleosides are different from the other nucleosides in said antisense compound.
14. The antisense compound of embodiment 13 wherein said differentially modified nucleosides are 2'-deoxynucleosides.
15. A method of enhancing the ability of a substituted or unsubstituted 2'-O-alkyl uniformly modified antisense compound to modulate the activity of a miRNA by incorporating into said antisense compound a plurality of bicyclic sugar modified nucleosides.
16. The method of embodiment 15 wherein wherein the 2'-substituent group each of said substituted or unsubstituted 2'-O-alkyl modified nucleosides is, independently, -O-(CH₂)ⱼ-CH₃, -O-(CH₂)₂-O-CH₃, -O(CH₂)₂-S-CH₃, O-(CH₂)₂-O-N(Rₘ)(Rₙ) or O-CH₂-C(=O)-N(Rₘ)(Rₙ), where j is 0, 1 or 2 and each Rₘ and Rₙ is, independently, H, an amino protecting group or substituted or unsubstituted C1-C10 alkyl.
17. The method of embodiment 15 wherein the bicyclic sugar of each bicyclic sugar modified nucleoside comprises a 2'-O-CH₂-4', or a 2'-O-(CH₂)₂-4' bridge.

## Claims

1. An antisense compound comprising a sequence of bicyclic sugar modified nucleosides, wherein said sequence is interrupted by the introduction of two or more regions comprising from 1 to 8 nucleosides having a different sugar moiety, wherein said compound comprises from 3 to 7 internal regions of bicyclic sugar modified nucleosides, for use in inhibiting microRNA activity.

2. The antisense compound of claim 1 wherein said different sugar moiety is a β-D-deoxynucleoside.

3. The antisense compound of claim 1 wherein said different sugar moiety is a substituted or unsubstituted 2'-O-alkyl nucleoside.

4. The antisense compound of claim 3 wherein each internal region comprises from 1 to 4 bicyclic sugar modified nucleosides.

5. The antisense compound of claim 3, wherein each of said substituted or unsubstituted 2'-O-alkyl modified nucleosides have the same sugar modification and each of said bicyclic sugar modified nucleosides have the same bicyclic modification.

6. The antisense compound of claim 3 or claim 5 wherein the 2'-substituent group each of said substituted or unsubstituted 2'-O-alkyl modified nucleosides is, independently, -O-(CH₂)ⱼ-CH₃, -O-(CH₂)₂-O-CH₃, -O(CH₂)₂-S-CH₃, O-(CH₂)₂-O-N(Rₘ)(Rₙ) or C-CH₂-C(=O)-N(Rₘ)(Rₙ), where j is 0, 1 or 2 and each Rₘ and Rₙ is, independently, H, an amino protecting group or substituted or unsubstituted C1-C10 alkyl.

7. The antisense compound of any preceding claim wherein the wherein the bicyclic sugar of each bicyclic sugar modified nucleoside comprises a 2'-O-CH₂-4', or a 2'-O-(CH₂)₂-4' bridge.

8. The antisense compound of claim 1 having one of the formulas:
A₅-B₁-A₅-B₁-A₄-B₁-A₆, (A-A-B)₇(-A)₂, (A-A-A-B)₅-A₃, A₅-B₁-A₂-B₁-A₂-B₁-A₂-B₁-A₁-B₁-A₃-B₁-A₂, A₃-B₃-A₂-B₃-A₂-B₃-A₇, A₃-B₃-A₂-B₃-A₂-B₃-A₂-B₃-A₂, A₃-B₂-A₂-B₃-A₂-B₂-A₈, A₅-B₂-A₂-B₃-A₂-B₃-A₅, wherein A is a substituted or unsubstituted 2'-O-alkyl modified nucleoside, B is a bicyclic sugar modified nucleoside, and each subscript number represents the number of repeats of the preceding nucleoside or block of nucleosides.

9. The antisense compound of claim 8 wherein each 2'-substituent group of said substituted or unsubstituted 2'-O-alkyl modified nucleosides is - O-(CH₂)₂-O-CH₃ and each bicyclic modified nucleoside comprises a 2'-O-CH₂-4' bridge.

10. The antisense compound of any preceding claim comprising from 15 to 30 linked nucleosides.

11. The antisense compound of any preceding claim, wherein each internucleoside linking group is, independently, a phosphodiester or a phosphorothioate.

12. The antisense compound of claim 11 further comprising a plurality of phosphorothioate internucleoside linkages.

13. The antisense compound of any preceding claim, wherein the antisense compound is complementary to miR-122a.

14. The antisense compound of any preceding claim, for use in lowering serum cholesterol.
